# EUROPEAN PATENT APPLICATION

(11) **EP 4 814 494 A1**
(43) Date of publication of application: **30.09.2026**
(21) Application number: 25315108.8
(22) Date of filing: 28.03.2025
(51) Int. Cl.: G01N 33/92

(54) **REGULATION OF SREBP ACTIVITY AND CHOLESTEROL FLUXES BY MODULATING CHOLESTEROL ESTER LEVEL**

(71) Applicant: Paris Sciences et Lettres, 75006 Paris (FR); Centre National de la Recherche Scientifique, 75016 Paris (FR); Université Paris Cité, 75006 Paris (FR); Sorbonne Université, 75006 Paris (FR)
(72) Inventor: Thaim, Abdou Rachid, 75005 Paris (FR); Zouiouich, Mehdi, 75005 Paris (FR); Omrane, Mohyeddine, 75005 Paris (FR)
(74) Representative: Cabinet Chaillot

(57) **Abstract**

Provided is a method for identifying molecules and compounds involved in the modulation of lipid synthesis and uptake, wherein said molecule or compound blocks or activates SREBP maturation, comprising contacting cells with a molecule or compound to be tested and observing the presence or absence of domains in the endoplasmic reticulum membrane formed by cholesterol esters with cholesterol or, alternately, localizing SREBP proteins within the cell after said contacting, wherein the molecule or compound is identified as an inhibitor of SREBP maturation when ER domains form within the cell or, alternately, SREBP signal is localized more in the endoplasmic reticulum, whereas the molecule or compound is identified as an activator of SREBP maturation when ER domains are absent or, alternately, SREBP signal is localized more in the nucleus or when its target genes are elevated.

## Description

### Background of the invention

The invention concerns a method for identifying molecules and compounds involved in the modulation of lipid synthesis and uptake. The molecules and compounds identified can be used to inhibit or activate the SREBP maturation, acting on cholesterol ester accumulation within the endoplasmic reticulum (ER) and cholesterol synthesis. The invention provides new products and applications in treating diseases, disorders, and deficiencies related to lipid metabolism, particularly cholesterol metabolism.

In most diseases, treatments to control cholesterol fluxes are needed to control cholesterol levels circulating the blood or in the liver or other organs, such as the brain, in the context of neurodegeneration. This can be achieved by controlling cholesterol efflux, influx, synthesis or degradation, or conversion at the cell level. All enzymes responsible for these mechanisms are under the control of SREBP. Therefore, identifying molecules that can modulate SREBP ER retention will enable the control of cholesterol fluxes.

SREBP (Sterol Regulatory Element-Binding Proteins) proteins belong to an essential pathway in cell metabolism and cell growth, which is dysregulated in many diseases. Targeting the activity of these proteins presents an interest in reducing the burden of frequent pathologies such as obesity, atherosclerosis, or cardiac diseases, or in improving the outcome of patients with certain types of cancer.

SREBP proteins are involved in de *novo* cholesterol synthesis and fatty acid synthesis by regulating the transcription of important proteins from these pathways. SREBPs are known to play a role in metabolic diseases and cancer, such as atherosclerosis, Metabolic dysfunction-associated steatotic liver disease (MASLD), type 2 diabetes or hepatocellular carcinoma.

Despite their important role, SREBP proteins are not commonly used as therapeutic target. SCAP (SREBP Cleavage-Activating Protein) protein binds to cholesterol and 25-hydroxycholesterol favors the binding of INSIG (Insulin-induced gene) proteins to SCAP, retaining the SCAP-SREBP complex into the endoplasmic reticulum. The direct modulation of the INSIG-SCAP-SREBP complex is a therapeutic approach already used by molecules such as fatostatin or betulin, but the ER retention system is not known to be targeted by drugs because of the lack of understanding of the mechanisms that govern SREBP ER retention and the difficulty to modulate protein-protein interaction.

So far, SREBPs activity can be modulated in various ways: by targeting the synthesis, the proteolytic activation of the proteins or the regulation of the nuclear form. Indirect targeting of SREBPs through the statins could lead to unwanted side effects. Therefore, the present inventors envisioned that modulation of the INSIG1-SCAP-SREBP complex migration (i.e. proteolytic activation) can be a way to regulate directly SREBP activity while limiting off-target effect to other pathways. There are currently only a few molecules known to specifically target this mechanism: sterols (or oxysterols, most notably 25-hydroxycholesterol), fatostatin (and its derivative FGH10019) and betulin.

Fatostatin is a non-steroidal molecule identified in a synthetic small molecule library as an inhibitor of insulin-induced adipogenesis in 3T3-L1 cells. It was later shown that fatostatin binds to SCAP and reduces the migration of SCAP-SREBP to the Golgi apparatus. This molecule showed moderate results in ob/ob mice model, and its low aqueous solubility led to the synthesis of derivatives such as FGH10019 with better outcome in the same mice model.

Betulin is a natural pentacyclic triterpene found in birch bark known to have antitumor activity. This molecule was shown to stimulate the binding of INSIG1 to SCAP, therefore reducing the maturation process of SREBPs. This activity could be due to a direct binding of betulin to SCAP. The same study showed that betulin can improve lipid parameters (triglycerides and cholesterol) in the blood and liver in an obese mice model and can even decrease the formation of atherosclerosis lesions as much as lovastatin.

There is, therefore, an ongoing need for alternative ways to regulate SREBP activity with limited side-effects, which are often observed with statins in the treatment of metabolic diseases and cancer.

### Description of the invention

The inventors presently show that cholesterol esters (CE) with cholesterol form domains in the endoplasmic reticulum (ER) membrane. These domains contain specific proteins, such as SCAP, a partner protein of SREBPs. The diffusion of SCAP in those domains is limited, trapping the protein and its binding partners within the ER membrane. Thus, SREBP-SCAP complex cannot be exported to the Golgi apparatus for cleavage, which limits SREBP function as a transcription factor.

By modulating the level of CE, they can regulate the activation level of SREBP. For instance, blocking ACAT proteins, which are enzymes synthesizing CE, induces an increase in SREBP activation/maturation. On the other hand, inhibiting NCEH1, which is responsible for CE hydrolysis, decreases SREBP activation/maturation.

The domains can be visualized in *cellulo* by swelling the cells with a hypotonic medium. Using this technique, the inventors show that the modulation of cholesterol ester (CE), which is important for forming ER domains, regulates the activation of the SREBP pathway.

They developed a method of identification of a molecule or compound acting on the maturation of SREBP proteins, either inhibitors or activators of said maturation.

The retention of the INSIG1-SCAP-SREBP complex in CE-rich domains opens a new rationale for drug treatment. Indeed, instead of directly targeting proteins from the SREBP complex, the inventors show that they can modulate the lipid composition of the ER membrane to induce or reduce the formation of domains that retain this protein complex. To do so, they propose to target enzymes (such as ACAT proteins or NCEH1) involved in the regulation of CE to modulate the CE level of the cells.

SREBP activity could be reduced, for instance, in diseases such as cancer or MASLD, by increasing the formation of these domains by using drugs inhibiting NCEH1 or by stimulating the activity of ACAT. Modulating the level of lipids to increase these ER domains broaden the therapeutic applications to block the maturation of SREBPs.

On the other hand, using an ACAT inhibitor such as avasimibe in addition to statins for dyslipidaemia could help treat patients resistant to statin alone treatment by increasing SREBP activation and thus increasing the expression of LDL-R. The synergy of the drug combination could potentially help to reduce the posology of statins and thus limit their side effects.

In the end, any means that increases cholesterol ester in the ER will inhibit lipid synthesis. Therefore, targeting any pathway, or combination of pathways, to promote CE accumulation will be a strategy to inhibit lipid biosynthesis. The opposite will lead to the triggering of lipid synthesis.

### Detailed description of the invention

The present invention relates to a method for identifying molecules and compounds involved in the modulation of lipid metabolism, in particular lipid synthesis and uptake, wherein said molecule or compound blocks or activates SREBP maturation, comprising contacting cells with a molecule or compound to be tested and observing the presence or absence of domains in the endoplasmic reticulum membrane, induced by the accumulation of phase-separating lipids such as cholesterol esters or, alternately, localizing SREBP proteins within the cell after said contacting, wherein the molecule or compound is identified as an inhibitor of SREBP maturation when ER domains form within the cell or, alternately, SREBP signal is localized more in the endoplasmic reticulum, whereas the molecule or compound is identified as an activator of SREBP maturation when ER domains are absent or, alternately, SREBP signal is localized more in the nucleus or when its target genes are elevated.

In a particular embodiment, the method comprises:
- contacting cells transfected with a fluorescent construct of one of the components of the complex INSIG-SCAP-SREBP or of SREBP regulator proteins such as ERLIN with a molecule or compound to be tested ;
- using fluorescence or brightfield imaging, or biochemical methods to verify the presence or absence of ER domains or, alternately, the localization of the complex proteins or SREBP regulator proteins such as ERLIN within the cell ;
- optionally, checking the protein expression of the target genes of SREBP ;
wherein the molecule or compound is identified as an inhibitor of SREBP maturation when ER domains form within the cell or, alternately, when SREBP signal is localized more in the endoplasmic reticulum, whereas the molecule or compound is identified as an activator of SREBP maturation when ER domains are absent within the cell or, alternately, when SREBP signal is localized more in the nucleus or when SREBP target genes are elevated.

In a particular embodiment, the method comprises the following steps :
- contacting cells with a molecule or compound to be tested ;
- swell the cells to identify the ER by (1) adding exogenous dyes and labeling it, or (2) prior to the previous step, having one of the SREBP regulators made fluorescently labelable (INSIGs, SCAP, SREBP, their regulator proteins such as ERLIN), which can be an endogenous tagging of the proteins or their overexpression, and/or (3) adding exogenous dyes that mark domains (e.g. DiI, Laurdan, solvatochromic dyes, etc.) ;
- using fluorescence or brightfield imaging to verify the presence or absence of ER domains within the cell or, alternately, the localization of the SREBP proteins within the cell (fluorescence, Raman or lipid/membrane imaging techniques).

The present invention also relates to a molecule or compound that modulates lipid metabolism, in particular lipid synthesis and uptake, wherein said molecule or compound is a modulator of SREBP trafficking to the nucleus via its action on the INSIG-SCAP-SREBP complex, the molecule or compound being an inhibitor of SREBP maturation when the INSIG-SCAP-SREBP complex is retained in the endoplasmic reticulum domains and the molecule or compound being an activator of SREBP maturation when it promotes the dissolution of domains and the INSIG-SCAP-SREBP complex by separation of SCAP-SREBP from INSIG.

According to a particular embodiment, the molecule or compound is an activator or inhibitor of an enzyme having cholesterol ester as a substrate or product, chosen from the group comprising ACAT (acyl-coenzymeA cholesterol acyltransferase), NCEH1 (Neutral Cholesterol Ester Hydrolase 1), or an enzyme chosen from the group comprising LDLR, HMGCR, SQLE, CH25H, HSL, DGATs, ACSL3, AGPAT.

Some other candidates includes, but are not limited to, Aster-GRAM, OSBP, SPT, CERT, Ceramidase, lipid and cholesterol transport proteins working at ER-organelle contacts such as atg2, VSP13, STARD3, and ER-shapin proteins which can alter domains such as reticulons, Atlastin.

According to another embodiment, the molecule or compound promotes the retention of the INSIG-SCAP-SREBP complex in the ER domains, such as cholesterol, 25-hydroxycholesterol, an inhibitor of serine hydrolase enzyme NCEH1 like JW480 (Sigma-Aldrich # SML0792), an inhibitor of DGATs like PF-06427878 (Sigma Aldrich # PZ0412), an inhibitor of DGAT1 or PF-06424439 (Sigma Aldrich # PZ0233), an inhibitor of DGAT2.

In yet another embodiment, the molecule or compound can promote SREBP maturation, such as avasimibe (Santa Cruz Biotechnology # sc-364315), an inhibitor of ACAT1, Sandoz 58-035 (Sigma-Aldrich # S9318), another ACAT inhibitor, or can also promote domain dissolution such as specific membrane lipids or neutral lipids including triacylglycerol.

According to another particular embodiment, the molecule or compound is an inhibitor of ER stress sensors or other lipid-modifying enzymes acting in the endoplasmic reticulum, such as Aster, ER stress proteins IRE1, ER-organelle contact mediators or other lipid transfer proteins such as Orps, ATG2, VPS-like proteins. These proteins will regulate cholesterol fluxes at the ER membrane and alter CE levels.

The present invention also relates to a pharmaceutical composition comprising:
- at least a molecule or compound as mentioned above ;
- a pharmaceutically acceptable excipient or carrier.

The pharmaceutically acceptable excipient or carrier is an excipient or carrier that does not produce an adverse, allergic, or other reaction when administered to an animal, preferably a human being. This includes any solvent, dispersion medium, coatings, antibacterial and antifungal agents, isotonic agents and absorption retarding agents, and the like. A pharmaceutically acceptable excipient or carrier refers to a non-toxic solid, semi-solid or liquid filler, diluent, encapsulating material or accessory formulation of any type. For human administration, the preparations must meet the requirements of sterility, pyrogenicity, general safety, and purity as required by the Food and Drug Safety Administration or European Medicines Agency.

According to a particular embodiment of the present invention, the pharmaceutical composition is for use in a method of treatment of diseases, disorders or deficiencies related to lipid metabolism, in particular diseases, disorders or deficiencies related to cholesterol metabolism.

The diseases, disorders or deficiencies related to lipid metabolism, are, for example, but not limited to, atherosclerosis, neurodegenerative diseases, Metabolic dysfunction-associated steatotic liver disease (MASLD), type 2 diabetes or cancers, such as hepatocellular carcinoma.

The present invention also relates to the use of a molecule or compound or pharmaceutical composition of the invention for increasing the retention of the INSIG-SCAP-SREBP complex within the endoplasmic reticulum via increasing the cholesterol ester accumulation within the ER and blocking the synthesis and uptake of cholesterol.

It also relates to the use of a molecule or compound or pharmaceutical composition of the invention for dissolving the INSIG-SCAP-SREBP complex, via decreasing the cholesterol ester accumulation within the ER and stimulating the synthesis and uptake of cholesterol.

The present invention also relates to a molecule or compound or pharmaceutical composition for use in the treatment of diseases, disorders or deficiencies related to lipid metabolism, in particular diseases, disorders or deficiencies related to cholesterol metabolism, said molecule or compound or pharmaceutical composition being able to increase the retention of the INSIG-SCAP-SREBP complex within the endoplasmic reticulum, via increasing the cholesterol ester accumulation within the ER and blocking the synthesis and uptake of cholesterol.

SREBP activity could be reduced, for instance, in diseases such as cancer, neurodegenerative disorders, or MASLD by increasing the formation of CE domains by using drugs inhibiting NCEH1 or by stimulating the activity of ACAT. Modulating the level of lipids to increase these ER domains broaden the therapeutic applications in order to block the maturation of SREBPs.

The invention also relates to a molecule or compound or pharmaceutical composition for use in the treatment of diseases, disorders or deficiencies related to lipid metabolism, in particular diseases, disorders or deficiencies related to cholesterol metabolism, said molecule or compound or pharmaceutical composition being able to induce the dissolution of the INSIG-SCAP-SREBP complex, via decreasing the cholesterol ester accumulation within the ER and stimulating the synthesis and uptake of cholesterol.

Stimulating cholesterol synthesis can be therapeutic in specific conditions where insufficient cholesterol or disrupted cholesterol metabolism leads to negative health consequences. It can be the case in neurodegenerative diseases such as Alzheimer's disease where there is evidence suggesting that low brain cholesterol might contribute to amyloid-beta plaque formation and neuronal dysfunction. Cholesterol is also the precursor for steroid hormones, so in conditions like adrenal insufficiency or hypogonadism, there is also a need to stimulate cholesterol synthesis. There are many other conditions which would benefit from a stimulation of the synthesis and uptake of cholesterol that would be apparent to the skilled person.

Moreover, using an ACAT inhibitor such as avasimibe in addition to statins for dyslipidaemia could help treat patients resistant to statin alone treatment by increasing SREBP activation and thus increasing the expression of LDL-R. The synergy of the drug combination could help reduce the posology of statins and thus limit their side effects. By achieving better lipid control, combination therapy can significantly reduce the risk of cardiovascular events such as heart attack, stroke, and coronary artery disease.

The present invention also relates to a method for increasing the cholesterol ester level within the endoplasmic reticulum, comprising:
- administering an effective amount of a molecule or compound - inhibitor of the SREBP maturation - or an effective amount of the pharmaceutical composition to a cell or GERV;
wherein the method results in an increase of the retention of the INSIG-SCAP-SREBP complex within the ER domains, thereby inhibiting the synthesis of cholesterol in the cell.

The giant organelle vesicles are obtained with the method disclosed in EP 21202428.5 and PCT application WO/2023/062149 where the giant organelle vesicles are obtained from the group of organelles consisting of the endoplasmic reticulum, mitochondria, lysosome, nucleus, Golgi apparatus, vacuole, chloroplast, autophagosome, autolysosomes, endosomes, plasma membranes, peroxisome, multivesicular bodies.

The term "giant organelle vesicles" (GOVs) means organelles having a mean size generally over 1 µm, from about 1 to 50 µm, preferably from about 2 to 15 µm, more preferably from about 3 to 10 µm, the most preferably of about 5 µm. Such giant organelles have a surface-to-volume ratio decreased compared to their native form which ranges from about 3 µm¹ to about 0,06 µm⁻¹, preferably from about 1,5 µm⁻¹ to about 0,15 µm⁻¹, more preferably from about 0,3 µm⁻¹ to about 1 µm⁻¹. Such giant organelles have a membrane tension above 10⁻⁶mN/m and below 10mN/m.

GERVs are giant organelles vesicles derived from the endoplasmic reticulum.

The present invention also relates to a method for decreasing the cholesterol ester level within the endoplasmic reticulum, comprising:
- administering an effective amount of a molecule or compound - activator of the SREBP maturation - or an effective amount of the pharmaceutical composition to a cell or GERV ;
wherein the method results in a dissolution of the INSIG-SCAP-SREBP complex within the ER, thereby activating the synthesis of cholesterol in the cell.

The present invention also relates to a method for generating ER domains in giant endoplasmic reticulum vesicles (GERVs), comprising the following steps:
- labelling one of the components of the INSIG-SCAP-SREBP complex or SREBP regulator proteins such as ERLIN ;
- contacting the cells with an activator of the formation of ER domains, such as an NCEH1 inhibitor or cholesterol or 25-hydroxycholesterol ;
- swelling said cells by incubating the cells with an hypotonic medium and identifying the ER by adding exogenous dyes ;
- swollen cells being observed by confocal microscopy or broken down to extract the GERVs,
wherein the ER domains allow the retention of the INSIG-SCAP-SREBP complex.

In a particular embodiment, the method for generating ER domains in giant endoplasmic reticulum vesicles (GERVs) comprises the following steps:
- transfecting the cells with a fluorescent construct of SCAP or INSIG1, for example; or having the endogenous proteins labelable for further fluorescence tagging (e.g. by knockin);
- contacting the transfected cells with an activator of the formation of ER domains, such as an NCEH1 inhibitor or cholesterol or 25-hydroxycholesterol;
- putting said cells in an hypotonic medium to allow cell swelling;
- swollen cells are observed by confocal microscopy or broken down to extract the GERVs,
wherein the ER domains allow the retention of the INSIG-SCAP-SREBP complex.

To better illustrate the subject of the present invention, a description is given below of non-limiting, illustrative examples in connection with the appended figures.

### Brief description of the figures

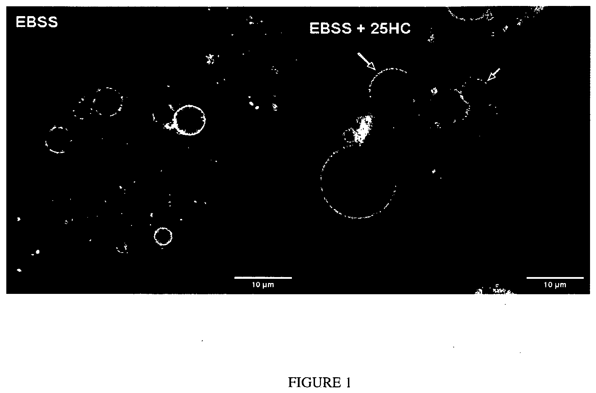

Figure 1 represents fluorescent confocal microscopy imaging of swollen Huh-7 cells following a hypotonic shock showing GFP-SCAP signal, either starved with EBSS (left panel) to deplete cholesterol or with addition of 25-hydroxycholesterol (right panel) before and during EBSS starvation. The arrows point to ER domains.

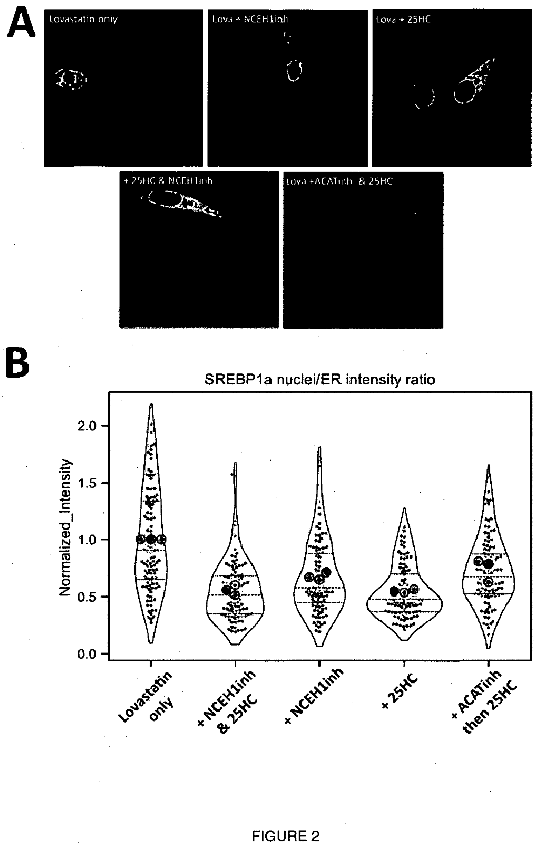

Figure 2A represents fluorescent confocal microscopy imaging of HeLa cells overexpressing GFP-SREBP1a subjected to a treatment with lovastatin for 4 hours. Some of the cells were treated with NCEH1 inhibitor (JW480), 25-hydroxycholesterol (25HC) for 2 hours before the addition of lovastatin. In the bottom right image, ACAT inhibitor (Sandoz 58-035) was added 1 hour prior the addition of 25-hydroxycholesterol.
Figure 2B represents the normalized intensity ratio of SREBPla in the nuclei over the the rest of the cell in the following conditions: lovastatin only, lovastatin + NCEH1 inhibitor & 25HC, lovastatin + NCEH1 inhibitor, lovastatin + 25 HC, and lovastatin + ACAT inhibitor & 25HC.

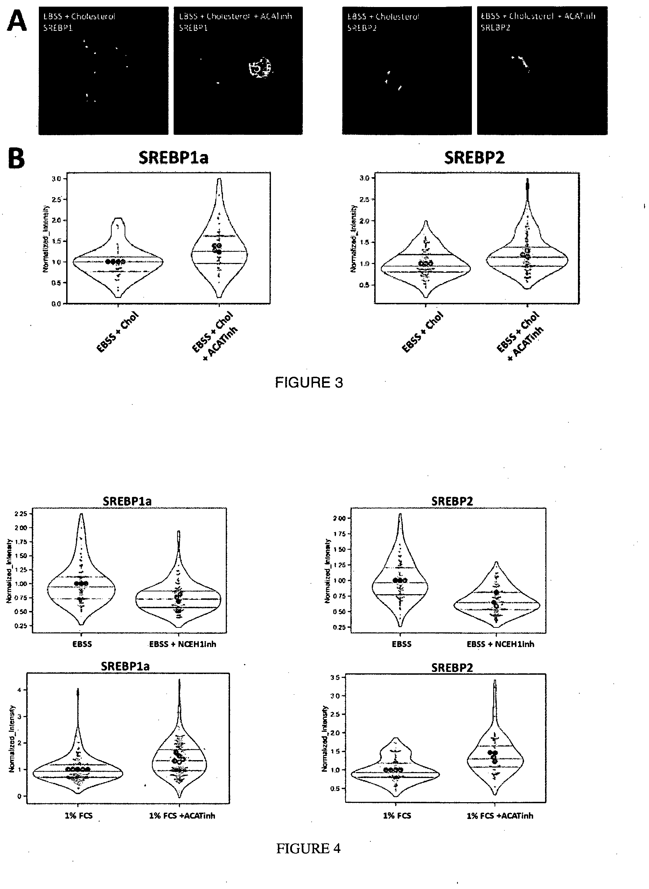

Figure 3A represents fluorescent confocal microscopy imaging of Huh-7 cells overexpressing GFP-SREBP1a (left panels) or GRP-SREBP2 (right panels) by transient transfection. Cells were starved in EBSS overnight and ACAT inhibitor (Sandoz 58-035) was added 2 hours prior the addition of cholesterol with which the cells were fed for 4 hours prior imaging.
Figure 3B represents the intensity of GFP-SREBP1a/2 normalized by cell by dividing the intensity of the protein in the nuclei versus the rest of the cell in the following conditions: EBSS + cholesterol or EBSS + cholesterol + ACAT inhibitor.
Figure 4 represents the intensity of GFP-SREBP1a/2 normalized by cell by dividing the intensity of the protein in the nuclei versus the cytoplamic signal (in Huh-7 cells) in the following conditions: EBSS or EBSS + NCEH1 inhibitor (JW480) in the top panels, 1% fetal calf serum (FCS) overnight or 1% FCS overnight + ACAT inhibitor (Sandoz 58-035) in the bottom panels.

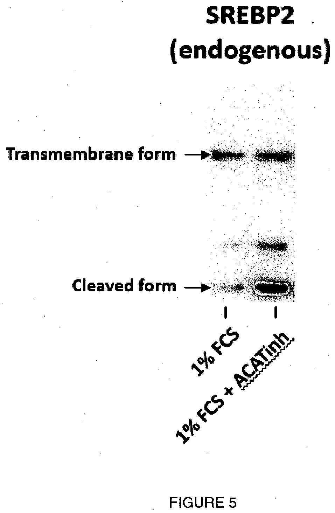

Figure 5 represents the Western blotting of endogenous SREBP2 protein in Huh-7 cells. Cells were grown in 1% fetal calf serum overnight with or without ACAT inhibitor (Sandoz 58-035).

### Examples

### Example 1: SCAP partitioning at the ER membrane is regulated by CE level

### Material and methods

Huh7 cells were plated on MatTek petri dishes (MatTek, P35G-1.5-14-C) and allowed to grow for 24h. 25-hydroxycholesterol was added to one condition for 2h at 2.5 µM in normal medium. Cells were then starved overnight in EBSS (ThermoFisher, 24010043), condition with prior 25HC was kept with the molecule during the starvation. Cells were swollen using an hypotonic medium (medium diluted 20 times) for 20 minutes before imaging.

Images were acquired on a Zeiss LSM 880 confocal microscope (63x, NA 1.4).

Figure 1 shows GFP-SCAP on swollen Huh7 cells using a hypotonic shock. Cells were starved using EBSS (left panel) to deplete the cholesterol and induce the activation of the SREBP pathway. GFP-SCAP signal is homogenous all around the ER vesicles. On the right panel, 25-hydroxycholesterol (25HC) was added before and during starvation to maintain the pool of CE. In that case, GFP-SCAP is partitioning in ER domains as shown by the heterogeneous signal (arrows).

These data show that by modulating CE level, the inventors were able to regulate the formation of ER domain and the localisation of SCAP.

### Example 2: Modulation of SREBP maturation by increasing CE level

### Material and methods

### Quantification of SREBPs activation by confocal imaging

Fifty thousand cells were plated in 12-well plates on glass coverslips. Cells were transfected with GFP-SREBP1a (truncated version of SREBP1a; missing first 30 amino acids) or GFP-SREBP2 with X-tremeGENE HP DNA (Roche, XTGHP-RO) the same day as the plating. Cells were grown in DMEM high glucose (ThermoFisher, 10569010) supplemented with either 1% FCS or 10% FCS depending on the experiments. Inhibitors were added for the indicated amount of time in the media in 50 µL of Opti-MEM (ThermoFisher, 11058021). 48 hours after transfection, cells were fixed in 4% PFA PBS and nuclei stained with Hoechst (ThermoFisher, H21486). Cells were mounted on glass slides in ProLong Gold (ThermoFisher, P36934). Images were acquired on a Zeiss LSM 880 confocal microscope (63x, NA 1.4).

For image processing, cell masks were obtained using CellPose locally. Then, images were analyzed on CellProfiler. A nuclei mask was obtained using an intensity-based thresholding method (Otsu). The intensity of the GFP (*i.e.* SREBPla or SREBP2) was measured in the nuclei mask and in the cytosol mask (*i.e*. cell mask minus nuclei mask). Data from CellProfiler were processed on Spyder 5.5.1 (Python 3.11.8) and results were normalized by the mean intensity of the control condition (*i.e.* without drug addition).

Figure 2 shows HeLa cells overexpressing GFP-SREBP1a (truncated version of SREBP1a; missing first 30 amino acids) by transient transfection. Cells were treated with NCEH1 inhibitor (JW480, Sigma-Aldrich, ref SML0792), 25-hydroxycholesterol (25HC) for 2 hours before addition of lovastatin for 4 hours. ACAT inhibitor (Sandoz 58-035, Sigma-Aldrich, ref S9318) was added 1 hour prior the addition of 25HC. The intensity of GFP-SREBP was normalized by cell by dividing the intensity of the protein in the nuclei versus the rest of the cell and shown in Figure 2B.

As seen on the microscopy image in Figure 2A, lovastatin alone led to a strong signal of SREBPla inside the nuclei, reflecting its maturation. However, when NCEH1 inhibitor, 25HC or a combination of both were added, the nuclei signal decreased. Blocking ACAT prior adding 25HC partially reversed the effect of 25HC, showing that 25HC stimulates CE synthesis, which plays a role in the maturation of SREBP1a.

All together, these data show that by modulating the CE level of the cells, the activation of SREBPla can be regulated.

Figure 3 shows Huh7 cells overexpressing GFP-SREBP1a (truncated version of SREBP1a; missing first 30 amino acids) (left) or GFP-SREBP2 (right) by transient transfection. Cells were starved in EBSS overnight. ACAT inhibitor (Sandoz 58-035) was added 2 hours prior the addition of cholesterol. Cells were fed with cholesterol for 4 hours prior imaging. The intensity of GFP-SREBP1a/2 was normalized by cell by dividing the intensity of the protein in the nuclei versus the rest of the cell.

As seen on microscope images, cholesterol addition inhibits the maturation of SREBPs (i.e. nuclei location). However, by blocking ACATs, the main enzymes producing sterol esters, SREBPs are still maturing and translocated to the nuclei (see quantifications and microscope images). These results demonstrate that sterol esters are major regulators of SREBPs activation.

Figure 4 shows the quantification of SREBP1a (truncated version of SREBP1a; missing first 30 amino acids) (left) or SREBP2 (right) activation by confocal imaging in Huh-7 cells (Japanese Collection of Research Bioresources #JCRB0403). On the top row, cells were starved overnight with EBSS, inducing the maturation of SREBPla and SREBP2. The addition of NCEH1 inhibitor (JW480) prior and during starvation mitigates the activation of both SREBPla and SREBP2 as seen by the decrease of intensity. These results thus show that SREBPs maturation can be blocked by increasing the sterol esters level.

On the bottom row, cells were grown in 1% FCS overnight, reducing the maturation of SREBPs proteins. However, the addition of ACAT inhibitor (Sandoz 58-035) prior and during 1% FCS increases SREBPs activation. In conclusion, SREBPs are regulated by sterol esters level. By targeting the main enzymes regulating cholesterol esters (i.e. ACAT and NCEH1), the inventors can modulate the maturation of both SREBPla and SREBP2.

### Quantification of SREBPs activation by Western Blot

Cells were seeded in 6-well plates and allowed to grow for 48h. Inhibitors were added for the indicated amount of time in the media in 50 µL of Opti-MEM. Cells were collected and washed with PBS, then lysed with RIPA buffer supplemented with complete (Sigma-Aldrich, 11697498001) for 20 min on ice. Lysates were loaded with SDS sample buffer and resolved by 4-10% SDS-PAGE and electrotransferred to PVDF sheets (Sigma-Aldrich, IPVH00010). The membrane was blocked in PBS containing 1% nonfat dry milk and 0.1% Tween 20 (Sigma-Aldrich, P9416) and then incubated with the primary antibody (anti-SREBP1a or anti-SREBP2 from Abcam, AB30682 and AB3259). After washing, the blots were incubated with the appropriate secondary antibodies conjugated with HRP. Protein-antibody complexes were visualized by an enhanced chemiluminescence detection system.

SREBPs activation can be then observed by measuring the intensity of the cleaved form and the transmembrane form on ImageJ.

Figure 5 shows a Western Blot of endogenous SREBP2 in Huh7 cells. Cells were grown in 1% FCS overnight with or without ACAT inhibitor (Sandoz 58-035). As seen on the blot, the inhibition of ACAT increases the matured form of SREBP2 at the endogenous level. These data confirm the results shown above with overexpressed SREBP2.

The examples given above are only preferred embodiments of the invention and are not intended to limit the scope of the present invention. Modifications, replacements by equivalents or improvements made by persons skilled in the art without departing from the spirit of the present invention must come within the framework of the present invention defined by the appended claims.

## Claims

1. - Method for identifying molecules and compounds involved in the modulation of lipid metabolism, in particular lipid synthesis and uptake, wherein said molecule or compound blocks or activates SREBP maturation, comprising contacting cells with a molecule or compound to be tested and observing the presence or absence of domains in the endoplasmic reticulum membrane, induced by the accumulation of phase-separating lipids such as cholesterol esters or, alternately, localizing SREBP proteins within the cell after said contacting,
wherein the molecule or compound is identified as an inhibitor of SREBP maturation when ER domains form within the cell or, alternately, SREBP signal is localized more in the endoplasmic reticulum, whereas the molecule or compound is identified as an activator of SREBP maturation when ER domains are absent or, alternately, SREBP signal is localized more in the nucleus or when its target genes are elevated.

2. - Method for identifying molecules and compounds involved in the modulation of lipid metabolism, in particular lipid synthesis and uptake according to claim 1, wherein said molecule or compound blocks or activates SREBP maturation, comprising:
- contacting cells transfected with a fluorescent construct of one of the components of the complex INSIG-SCAP-SREBP or of SREBP regulator proteins such as ERLIN with a molecule or compound to be tested ;
- using fluorescence or brightfield imaging, or biochemical methods to verify the presence or absence of ER domains or, alternately, the localization of the complex proteins or SREBP regulator proteins such as ERLIN within the cell ;
- optionally, checking the protein expression of the target genes of SREBP ;
wherein the molecule or compound is identified as an inhibitor of SREBP maturation when ER domains form within the cell or, alternately, when SREBP signal is localized more in the endoplasmic reticulum, whereas the molecule or compound is identified as an activator of SREBP maturation when ER domains are absent within the cell or, alternately, when SREBP signal is localized more in the nucleus or when SREBP target genes are elevated.

3. - Molecule or compound modulates lipid metabolism, in particular lipid synthesis and uptake as identified according to any of claims 1 or 2, wherein said molecule or compound is a modulator of SREBP trafficking to the nucleus via its action on the INSIG-SCAP-SREBP complex, the molecule or compound being an inhibitor of SREBP maturation when the INSIG-SCAP-SREBP complex is retained in the endoplasmic reticulum domains and the molecule or compound being an activator of SREBP maturation when it promotes the dissolution of domains and the INSIG-SCAP-SREBP complex by separation of SCAP-SREBP from INSIG.

4. - Molecule or compound according to claim 3, wherein said molecule or compound is an activator or inhibitor of an enzyme having cholesterol ester as a substrate or product, chosen from the group comprising ACAT (acyl-coenzymeA cholesterol acyltransferase), NCEH1 (Neutral Cholesterol Ester Hydrolase 1), or an enzyme chosen from the group comprising LDLR, HMGCR, SQLE, CH25H, HSL, DGATs, ACSL3, AGPAT.

5. - Molecule or compound according to claim 3, wherein said molecule or compound promotes the retention of the INSIG-SCAP-SREBP complex in the ER domains, such as cholesterol, 25-hydroxycholesterol, an inhibitor of serine hydrolase enzyme NCEH1 like JW480, an inhibitor of DGATs like PF-06427878, an inhibitor of DGAT1, or PF-06424439, an inhibitor of DGAT2, or wherein said molecule or compound promotes SREBP maturation, such as avasimibe, an inhibitor of ACAT1, Sandoz 58-035, another ACAT inhibitor, or wherein said molecule or compound promotes domain dissolution such as specific membrane lipids or neutral lipids including triacylglycerol.

6. - Molecule or compound according to claim 3, wherein said molecule or compound is an inhibitor of ER stress sensors or other lipid-modifying enzymes acting in the endoplasmic reticulum, such as Aster, ER stress proteins IRE1, ER-organelle contact mediators or other lipid transfer proteins such as Orps, ATG2, VPS-like proteins.

7. - Pharmaceutical composition comprising:
- at least a molecule or compound according to any of claims 3-6;
- a pharmaceutically acceptable excipient or carrier.

8. - Pharmaceutical composition according to claim 7 for use in a method of treatment of diseases, disorders, or deficiencies related to lipid metabolism, in particular diseases, disorders, or deficiencies related to cholesterol metabolism.

9. - Use of a molecule or compound according to any of claims 3-6 or pharmaceutical composition according to claims 7-8 for increasing the retention of the INSIG-SCAP-SREBP complex within the endoplasmic reticulum, via increasing the cholesterol ester accumulation within the ER and blocking the synthesis and uptake of cholesterol.

10. - Use of a molecule or compound according to any of claims 3-6 or pharmaceutical composition according to claims 7-8 for dissolving the INSIG-SCAP-SREBP complex, via decreasing the cholesterol ester accumulation within the ER and stimulating the synthesis and uptake of cholesterol.

11. - Molecule or compound or pharmaceutical composition for use in the treatment of diseases, disorders or deficiencies related to lipid metabolism, in particular diseases, disorders or deficiencies related to cholesterol metabolism, wherein said molecule or compound is as defined in any of claim 3-6 or said pharmaceutical composition is as defined in any of claims 7-8, and wherein said molecule or compound or pharmaceutical composition is able to increase the retention of the INSIG-SCAP-SREBP complex within the endoplasmic reticulum, via increasing the cholesterol ester accumulation within the ER and blocking the synthesis and uptake of cholesterol.

12. - Molecule or compound or pharmaceutical composition for use in the treatment of diseases, disorders or deficiencies related to lipid metabolism, in particular diseases, disorders or deficiencies related to cholesterol metabolism, wherein said molecule or compound is as defined in any of claim 3-6 or said pharmaceutical composition is as defined in any of claims 7-8, and wherein said molecule or compound or pharmaceutical composition is able to induce the dissolution of the INSIG-SCAP-SREBP complex, via decreasing the cholesterol ester accumulation within the ER and stimulating the synthesis and uptake of cholesterol.

13. - Method for increasing the cholesterol ester level within the endoplasmic reticulum, comprising:
- administering an effective amount of a molecule or compound - inhibitor of the SREBP maturation - of any of claims 3-6 or an effective amount of the pharmaceutical composition of any of claims 7-8 to a cell or GERV,
wherein the method results in an increase of the retention of the INSIG-SCAP-SREBP complex within ER domains, thereby inhibiting the synthesis of cholesterol in the cell.

14. - Method for decreasing the cholesterol ester level within the endoplasmic reticulum, comprising:
- administering an effective amount of a molecule or compound - activator of the SREBP maturation - of any of claims 3-6 or an effective amount of the pharmaceutical composition of any of claims 7-8 to a cell or GERV,
wherein the method results in a dissolution of the INSIG-SCAP-SREBP complex within the ER, thereby activating the synthesis of cholesterol in the cell.

15. - Method for generating ER domains in giant endoplasmic reticulum vesicles (GERVs), comprising the following steps :
- labelling one of the components of the INSIG-SCAP-SREBP complex or SREBP regulator proteins such as ERLIN ;
- contacting the cells with an activator of the formation of ER domains, such as an NCEH1 inhibitor or cholesterol or 25-hydroxycholesterol ;
- swelling said cells by incubating the cells with an hypotonic medium and identifying the ER by adding exogenous dyes ;
- swollen cells being observed by confocal microscopy or broken down to extract the GERVs,
wherein the ER domains allow the retention of the INSIG-SCAP-SREBP complex.
